(19) Europäisches Patentamt
European Patent Office
Office européen des brevets

(11) Publication number: 0 347 235
A2

(12) # EUROPEAN PATENT APPLICATION

(21) Application number: 89306098.8

(22) Date of filing: 15.06.89

(51) Int. Cl.⁴: G 03 C 7/38
C 07 D 487/04
//(C07D487/04,249:00,231:00)

(30) Priority: 15.06.88 JP 148485/88
28.06.88 JP 160277/88
28.06.88 JP 160278/88

(43) Date of publication of application:
20.12.89 Bulletin 89/51

(84) Designated Contracting States: DE GB

(71) Applicant: KONICA CORPORATION
No. 26-2, Nishishinjuku 1-chome Shinjuku-ku
Tokyo (JP)

(72) Inventor: Miura, Norio
Konica Corporation 1 Sakura-machi
Hino-shi Tokyo (JP)

Wada, Hajime
Konica Corporation 1 Sakura-machi
Hino-shi Tokyo (JP)

Ishii, Fumio c/o Konica Corpporation
1 Sakura-machi, Hino-shi
Tokyo (JP)

Kida, Shuji c/o Konica Corporation
1 Sakura-machi, Hino-shi
Tokyo (JP)

(74) Representative: Ellis-Jones, Patrick George Armine et al
J.A. KEMP & CO. 14 South Square Gray's Inn
London WC1R 5EU (GB)

(54) **Photosensitive silver halide color photographic material.**

(57) A photosensitive silver halide color photographic material is disclosed which has a good color forming property and sufficient resistance to formalin and ensures stability of the color image density to change in pH of the developer solution and good light fastness of the image formed therein. The photographic material comprises a silver halide emulsion layer which contains a magenta coupler representd by the following formula:

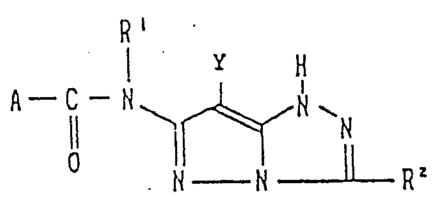

wherein $R^1$ represents a hydrogen atom, alkyl group, acyl group, or aryl group, $R^2$ a hydrogen atom or a substituent group, Y - O - $R^3$, - S - $R^{13}$ or a chlorine, bromine or iodine atom wherein $R^3$ represents an aryl group whether substituted and $R^{13}$ an aryl, an alkyl or heterocyclic group whether substituted or not substituted, and A an aryl group whether substituted or not substituted.

EP 0 347 235 A2

**Description**

**PHOTOSENSITIVE SILVER HALIDE COLOR PHOTOGRAPHIC MATERIAL**

## BACKGROUND OF INVENTION

The present invention relates to a photosensitive silver halide color photographic material, and more particularly to one containing a magenta coupler.

As couplers used in photosensitive silver halide color photographic materials (hereinafter referred to as "color photosensitive material"), it is known that, in general, use is made of open-chain Ketomethylene compounds for yellow couplers, pyrazolone compounds and pyrazoloazole compounds for magenta couplers, and phenol compounds and naphthol compounds for cyan couplers.

For magenta couplers, pyrazolone compounds have been in wide use, some known examples being those in the disclosures of U.S. Patents Nos. 2,600,788 and 3,519,429, and Japanese Patent Publication Open to Public Inspection (hereinafter referred to as "Japanese Patent O.P.I. Publication") Nos. 49-111631/1974 and 57-35858/1982. The use of pyrazolone compounds for magenta couplers, however, involves undesirable secondary absorption in the dyes formed thereby as pointed out in The Theory of the Photographic Process (4th Ed.), Macmillan (1977), Pages 356-358, Fine Chemical (Vol. 14, No. 8), C.M.C., Pages 38-41, and the Substances of Lectures by the Photographic Society of Japan at the 1985 Annual Meeting (at Shigaku Kaikan on May 23 and 24, 1985), Pages 103-110. A solution of this problem has been awaited.

On the other hand, secondary absorption does not occur in the dyes formed by magenta couplers of pyrazoloazole compounds, as noted in the above-mentioned literature; pyrazoloazole compounds are credited with advantages as magenta couplers also in the disclosures of U.S. Patents Nos. 3,725,067, 3,758,309 and 3,810,761, etc., besides the above-mentioned literature. Despite these applauses, such pyrazoloazole couplers have defects in that their color forming property and resistance to formalin are inadequate, that even a slight deviation in pH of the developer solution causes the density of the color image to change greatly, and that the images formed are inferior in light fastness.

To improve on such pyrazoloazole compounds with respect to color forming property and resistance to formalin, there has been development of couplers, which are described in Japanese Patent O.P.I. Publication No. 62-205348/1987, which, however, have defects in that the density of the color image is greatly affected by changes in pH of the developer solution, and the light fastness of the developed image is inferior. The couplers described in Japanese Patent Examined Publication No. 46-43947/1971, and Japanese Patent O.P.I. Publication Nos. 62-92944/1987 and 62-92946/1987 can not meet the requirement of the practical use with respect to color forming property. The compounds based on pyrazolo[3,2-c]-s-triazole, having a cyano group and methoxycarbonyl group at the active site, which is described in Japanese Patent O.P.I. Publication No. 62-195366/1987, correspond to an intermediate substance for the synthesis of the couplers embodying this invention, which will be dealt with later, and can hardly be said to have a color forming property.

## SUMMARY OF THE INVENTION

The invention has for its object to provide a photosensitive silver halide color photographic material which has a good color forming property and sufficient resistance to formalin and ensures stability of the color image density to changes in pH of the developer solution and good light fastness of the images formed therein. In accomplishing this object, according to this invention, there is provided a color photosensitive material which has one or more layers of silver halide emulsion on the support and contains in at least one layer of said layers of silver halide emulsion a magenta coupler represented by a formula

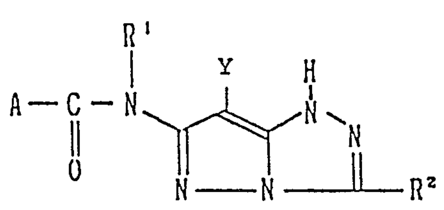

wherein $R^1$ represents a hydrogen atom, alkyl group, acyl group, or aryl group, $R^2$ a hydrogen atom or a substituent group, Y - O - $R^3$, - S - $R^{13}$ or a chlorine, bromine or iodine atom wherein $R^3$ represents an aryl group whether substituted and $R^{13}$ an aryl, an alkyl or heterocyclic group whether substituted or not substituted, and A an aryl group whether substituted or not substituted.

Detailed Description of the invention

A magenta coupler represented by the aforementioned formula [I] (hereinafter referred to as "coupler of this invention" or "compound of this invention") is contains a group

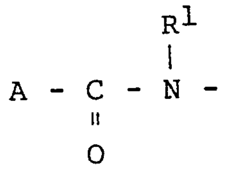

at the 6-position and a group - O - $R^3$, - S - $R^{13}$ or a chlorine, bromine or iodine atom at the 7-position in its structure.

An alkyl group represented by $R^1$ in the aforementioned formula [I] is one with a carbon number of 1-18, examples of such alkyl groups being methyl, ethyl, n-propyl, isopropyl, n-butyl, isobutyl, pentyl, hexyl, heptyl, octyl, dodecyl, and octadecyl; such an alkyl group may have a substituent group.

An acyl group represented by $R^1$ is one such as acetyl, propionyl, butyryl, isobutyryl, valeryl, isovaleryl and pivaloyl.

As an aryl group represented by $R^1$ phenyl or naphthyl is useful; each of these aryl groups having a substituent group is also useful.

As an alkyl group represented by $R^{13}$ is one with a carbon number of 1-22, examples of such alkyl groups being methyl, ethyl, n-propyl, isopropyl, n-butyl, isobutyl, t-butyl, undecyl, tridecyl, pentadecyl, heptadecyl eicosyl and dodecyl; such as alkyl group may have a substituent group.

As an aryl group represented by $R^3$, $R^{13}$ and A the presence of phenyl or naphthyl is desirable; each of these aryl groups having a substituent group is also useful.

A heterocyclic group represented by $R^{13}$ is a 5 or 6-membered ring containing a nitrogen atom or its condensed ring, an example of which is 2-pyridyl, 4-pyridyl, 2-thienyl, 2-furyl, 2-imidazolyl, pyrazinyl, 2-pyrimidinyl, 1-imidazolyl, 1-indolyl, phthalimide, 1,3,4-thiadiazole-2-yl, benzooxazole-2-yl, 2-quinolinyl, 2,4-dioxo-1,3-imidazolidyn-5-yl, 1-phenyltetrazolyl, succinimide, phthalimide and 1,2,4-triazole-2-yl.

Examples of a substituent group which may be introduced into an alkyl group or aryl group represented by $R^1$ or into an alkyl, aryl or heteroocyclic group represented by $R^3$, $R^{13}$, or A are halogen atoms (e.g., fluorine, chlorine and bromine), and groups known as alkyl, aryl, heterocyclic, nitro, cyano, alkoxy, aryloxy, alkylthio, arylthio, keto, sulfonamido, sulfamoyl, acylamino, carbamoyl, sulfonyl, sulfinyl, hydroxy, carboxy, amino, primary amino and secondary amino.

Examples of a group represented by $R^2$ are halogen atoms, groups known as alkyl, cycloalkyl, alkenyl, cycloalkenyl, alkinyl, aryl, heterocyclic aromatic, and phosphonyl, groups which bond through a carbonyl group, such as acyl, carbamoyl, alkoxycarbonyl and aryloxycarbonyl, and groups which bond through a heteroatom, specifically, such as sulfonyl, sulfinyl, sulfamoyl, alkylthio, arylthio, and heterocyclic aromaticthio (those which bond through a sulfur atom), such as alkoxy, aryloxy, heterocyclic aromatic-oxy, acyloxy and carbamoyloxy (bonding through an oxygen atom), and such as amino, acylamino, sulfonamido, imido, ureido, sulfamoylamino, alkoxycarbonylamino and aryloxycarbonylamino (bonding through a nitrogen atom).

An alkyl group represented by $R^2$ is one with a carbon number of 1-32, an alkenyl group and an alkinyl group for $R^2$ are respectively ones with a carbon number of 2-32, and a cycloalkyl group and a cycloalkenyl group for $R^2$ are respectively ones with a carbon number of 3-12, or preferably with a carbon number of 5-7. An alkyl group, alkenyl group or alkinyl group for $R^2$ can be of the straight chain as well as the branched chain.

Any of the above-mentioned groups (alkyl, alkenyl, alkinyl, cycloalkyl or cycloalkenyl) for $R^2$ may also have a substituent group; examples of substituents are arly group, cyano group, halogen atoms, heterocycles, cycloalkyl group, cycloalkenyl group, spiro-compound residue and organic hydrocarbon compound residue, groups which bond through a carbonyl group, such as acyl, carboxy, carbamoyl, alkoxycarbonyl, and aryloxycarbonyl, and groups bonding through a heteroatom, specifically, such as hydroxy, alkoxy, aryloxy, heterocyclicoxy, siloxy, acyloxy and carbamoyloxy (through an oxygen atom), such as nitro, amino (including dialkylamino etc.), sulfamoylamino, alkoxycarbonylamino, aryloxycarbonylamino, acylamino, sulfonamido, imido and ureido (through a nitrogen atom), and such as alkylthio, arylthio, heterocyclic-thio, sulfonyl, sulfinyl and sulfamoyl (through a sulfur atom(s)).

Examples of such groups represented by $R^2$, are groups known as methyl, ethyl, isopropyl, t-butyl, pentadecyl, heptadecyl, 1-hexylnonyl, 1,1′- dipentylnonyl, 2-chloro-t-butyl, trifluoromethyl, 1-ethoxytridecyl, 1-methoxyisopropyl, methanesulfonylethyl, 2,4-di-t-amylphenoxymethyl, anilino, 1-phenylisopropyl, 3-m-butanesulfonaminophenoxypropyl, 3-4′-[α-{4″(p-hydroxybenzenesulfonyl)phenoxy}dodecanoylamino] phenylpropyl, 3-[4′-α{-(2″,4″-di-t-amylphenoxy)butanamido}phenyl]-propyl, 4-[α-(o-chlorophenoxy)tetradecanamidophenoxy]propyl, allyl, cyclopentyl and cyclohexyl.

As an aryl group it is desirable for $R^2$ to have a phenyl group; this aryl group may have a substituent group (e.g., alkyl group, alkoxy group, or acylamino group).

Examples of the phenyl group for $R^2$ are groups known as phenyl, 4-t-butylphenyl, 2,4-di-t-amylphenyl, 4-tetradecanamidophenyl, hexadesiloxyphenyl and 4′-[α-(4″-t-butylphenoxy)tetradecanamido]phenyl.

As a heterocyclic group represented by $R^2$ group of a 5- to 7-membered ring is desirable; $R^2$ may have such a heterocyclic group in the condensed form as well as in the substituted form. Examples of such heterocyclic

groups are 2-furyl, 2-thienyl, 2-pyrimidinyl and 2-benzothiazolyl.

Examples of an acyl group represeted by $R^2$ are alkylcarbonyl groups, such as groups known as acetyl, phenylacetyl, dodecanoyl and α-2,4-di-t-amylphenoxybutanoyl, and arylcarbonyl groups, such as groups known as benzoyl, 3-pentadecyloxybenzoyl and p-chlorobenzoyl.

Examples of a sulfonyl group represeted by $R^2$ are alkylsulfonyls, such as methylsulfonyl and dodecylsulfonyl, and arylsulfonyls, such as benzenesulfonyl and p-toluenesulfonyl.

Examples of sulfinyl group represeted by $R^2$ are alkylsulfinyls, such as ethylsulfinyl, octylsulfinyl and 3-phenoxybutylsulfinyl, and arylsulfinyls, such as phenylsulfinyl and m-pentadecylphenylsulfinyl.

Examples of a phosphonyl group represeted by $R^2$ ar are alkylphosphonyls, such as butyloctylphosphonyl, alkoxyphosphonyls, such as octyloxyphosphonyl, aryloxyphosphonyl, such as phenoxyphosphonyl, and arylphosphonyl, such as phenylphosphonyl.

A carbamoyl group represeted by $R^2$ can be one in the substituted form with an alkyl group, aryl group (preferably a phenyl group), or the like, as a substituent.

Examples of a carbamoyl group for $R^2$ are N-methylcarbamoyl, N,N-dibutylcarbamoyl, N-(2-pentadecyloctylethyl), carbamoyl, N-ethyl-N-dodecylcarbamoyl and N-[3-(2,4-di-t-amylphenoxy)propyl]carbamoyl.

A sulfamoyl group represeted by $R^2$ can be one in the substituted form with an alkyl group, aryl group (preferably a phenyl group), or the like as a substituent.

Examples of a sulfamoyl group for $R^2$ are N-propylsulfamoyl, N,N-diethylsulfamoyl, N-(2-pentadecyloxyethyl)sulfamoyl, N-ethyl-N-dodecylsulfamoyl and N-phenylsulfamoyl.

A spiro-compound residue represeted by $R^2$ can be typified by spiro[3,3]heptan-1-yl.

An organic hydrocarbon compound residue represeted by $R^2$ can be typified by bicyclo[2,2,1]heptan-1-yl, tricyclo[3,3,1,1]decan-1-yl and 7,7-dimethyl-bicyclo[2,2,1]heptan-1-yl.

An alkoxy group represeted by $R^2$ may further have a substituent group from the groups hereinbefore referred to as substituents for an alkyl group. Examples of such alkoxy groups are groups known as methoxy, propoxy, 2-ethoxyethoxy, pentadecyloxy, 2-dodecyloxyethoxy and phenethyloxyethoxy.

As an aryloxy group represeted by $R^2$ phenyloxy is desirable. The aryl nucleus can further be substituted by any of groups or atoms for an aryl group which are hereinbefore referred to. Examples of such aryloxy groups are groups known as phenoxy, p-t-butylphenoxy, and m-pentadecylphenoxy.

As a heterocyclic-oxy group represeted by $R^2$ a 5- to 7-membered heterocycle is desirable; the heterocycle may further have a substituent group; examples are 3,4,5,6-tetrahydropyranyl-2-oxy and 1-phenyltetrazol-5-oxy.

Examples of an acyloxy group represeted by $R^2$ are alkylcarbonyloxy and arylcarbonyloxy. An acyloxy group for $R^2$ may further have a substituent group, examples being acetyloxy group, α-chloroacetyloxy group, and benzoyloxy group.

A carbamoyloxy group represeted by $R^2$ have a substituent group such as an alkyl group or aryl group, examples being N-ethylcarbamoyloxy group, N,N-diethylcarbamoyloxy group, and N-phenylcarbamoyloxy group.

An amino group represeted by $R^2$ may have a substituent group such as an alkyl group or aryl group (preferably a phenyl group), examples being groups known as ethylamino, anilino, m-chloroanilino, 3-pentadecyloxycarbonylanilino and 2-chloro-5-hexadecanamidoanilido.

Examples of an acylamino group represeted by $R^2$ are alkylcarbonylamino group and arylcarbonylamino group (preferably phenylcarbonylamino group). An acylamino group for $R^2$ may further have a substituent group, examples being groups known as acetoamido, α-ethylpropaneamido, N-phenylacetoamido, dodecaneamido, 2,4-di-t-amylphenoxyacetoamido and α-3-t-butyl-4-hydroxyphenoxybutaneamido.

Examples of a sulfonamido group represented by are alkylsulfonylamino group and arylsulfonylamino group. A sulfonamido group may further have a substituent group, examples being groups known as methylsulfonylamino,pentadecylsulfonylamino, benzensulfonamido, p-toluenesulfonamido and 2-methoxy-5-t-amylbenzensulfonamido.

An imido group represeted by $R^2$ may be of the open chain type as well as the ring type and may have a substituent group, examples being groups known as succinimido, 3-heptadecylsuccinimido, phthalimido and glutarimido.

An ureido group represeted by $R^2$ have a substituent group such as an alkyl group or aryl group (preferably phenyl group), examples being groups known as N-ethylureido, N-methyl-N-decylureido, N-phenylureido and N-p-tolylureido.

A sulfamoylamino group represeted by $R^2$ may have a substituent group such as an alkyl group or aryl group (preferably phenyl group), examples being N,N-dibutylsulfamoylamino group, N-methylsulfamoylamino group and N-phenylsulfamoylamino group.

An alkoxycarbonylamino group represeted by $R^2$ may further have a substituent group, examples being methoxycarbonylamino group, methoxyethoxycarbonylamino group and octadecyloxycarbonylamino group.

An aryloxycarbonylamino group represeted by $R^2$ may have a substituent group, examples being phenoxycarbonylamino group and 4-methylphenoxycarbonylamino group.

An alkoxycarbonyl group represeted by $R^2$ may further have a substituent group, examples being groups known as methoxycarbonyl, butyloxycarbonyl, dodecyloxycarbonyl, octadecyloxycarbonyl, ethoxymethoxycarbonyloxy and benziloxycarbonyl.

An aryloxycarbonyl group represeted by $R^2$ may further have a substituent group, examples being

phenoxycarbonyl group, p-chlorophenoxycarbonyl group and m-pentadecyloxyphenoxycarbonyl group.

An alkylthio group represeted by $R^2$ may further have a substituent group, examples being groups known as ethylthio, dodecylthio, octadecylthio, phenethylthio and 3-phenoxypropylthio.

As an arylthio group represeted by $R^2$ a phenylthio group is desirable; such an arylthio group may further have a substituent group; examples are groups known as phenylthio, p-methoxyphenylthio, 2-t-octhylphenylthio, 3-octadecylphenylthio, 2-carboxyphenylthio, and p-acetoaminophenylthio.

As a heterocyclicthio group represented by $R^2$ preferable is a 5 - 7 membered heterocyclicthio group, which may have a condensed ring or substituent group. Example of the group is 2-pyridylthio, 2-benzothiazolylthio and 2,4-diphenoxy-1,3,5-triazole-6-thio.

X represents a chlorine, bromine or iodide atom, in which the most preferable is a chlorine atom.

Hereunder are shown examples typifying the compounds employed as magenta couplers as represented in the formula [I] shown hereinbefore. They may be rewritten in formula [II] to [IV].

Examples of Compounds embodying the present invention

Formula I

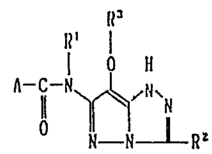

| No. | A | $R^1$ | $R^2$ | $R^3$ |
|---|---|---|---|---|
| 1 | 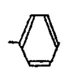 | -H | $-(CH_2)_3SO_2C_{12}H_{25}$ | 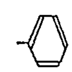 |
| 2 | 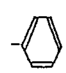 | -H | $-CH_3$ | 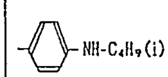 |
| 3 | 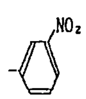 | -H | 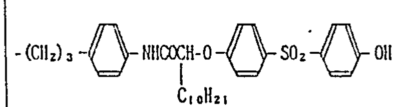 | 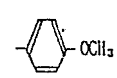 |
| 4 | 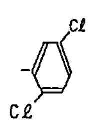 | -H | 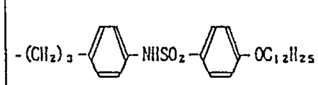 | 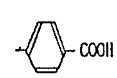 |
| 5 | 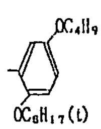 | $-CH_3$ | $-SCH_3$ | 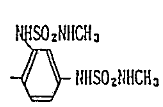 |
| 6 | 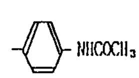 | -H | 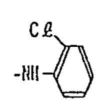 | 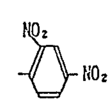 |

| No. | A | $R^1$ | $R^2$ | $R^3$ |
|---|---|---|---|---|
| 7 | 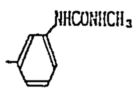 | -H | 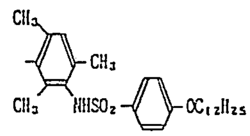 | 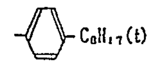 |
| 8 | 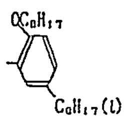 | -H | $-C_{15}H_{31}$ | 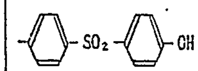 |
| 9 | 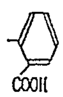 | -H | 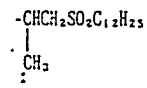 | 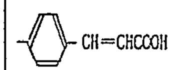 |
| 10 | 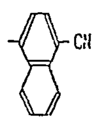 | -H | 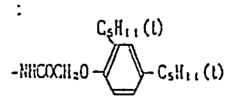 | 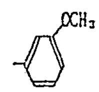 |
| 11 | 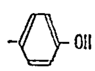 | $-COCH_3$ | 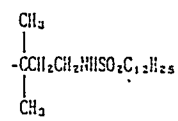 | 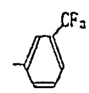 |
| 12 | 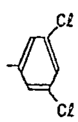 | 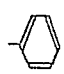 | 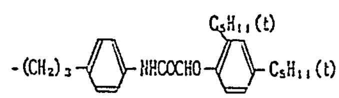 | 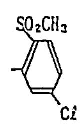 |
| 13 | 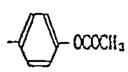 | 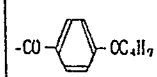 | $-SC_{12}H_{25}$ | 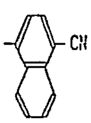 |

| No. | A | $R^1$ | $R^2$ | $R^3$ |
|---|---|---|---|---|
| 14 | 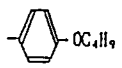 | -H | 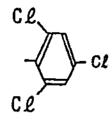 | 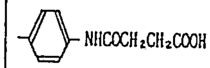 |
| 15 | 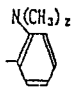 | -H | 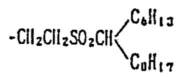 | 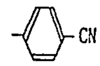 |
| 16 | 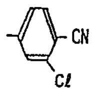 | -H | 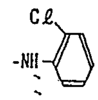 | 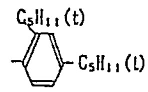 |
| 17 | 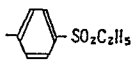 | -H | $-SCH_2CH_2COOH$ | 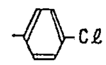 |
| 18 | 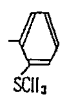 | -H | 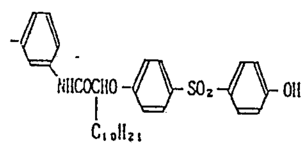 | 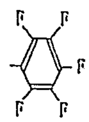 |
| 19 | 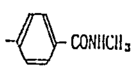 | $-C_2H_5$ | 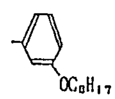 | 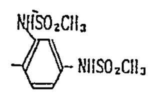 |
| 20 | 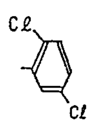 | -H | 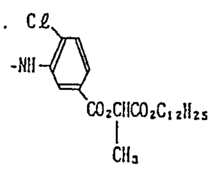 | 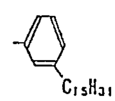 |
| 21 | 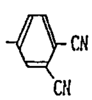 | -H | 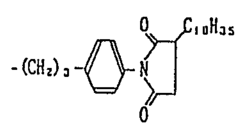 | 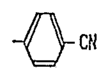 |

| No. | A | $R^1$ | $R^2$ | $R^3$ |
|---|---|---|---|---|
| 22 | 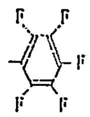 | -H | $-C_2H_5$ | 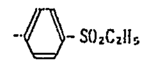 |
| 23 | 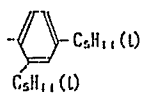 | 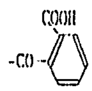 | 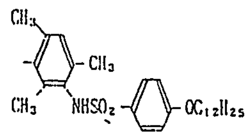 | 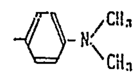 |
| 24 | 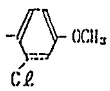 | -H | 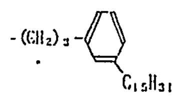 | 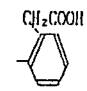 |
| 25 | 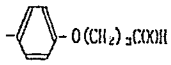 | -H | 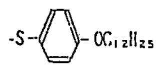 | 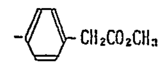 |
| 26 | 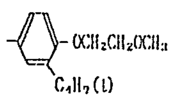 | -H | 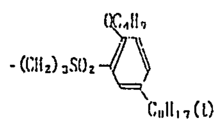 | 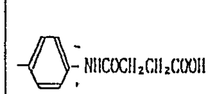 |
| 27 | 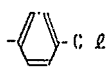 | -H | 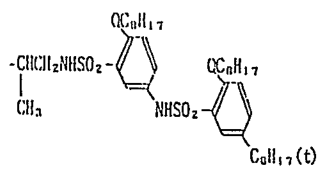 | 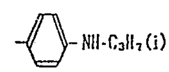 |
| 28 | 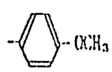 | -H | 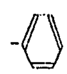 | 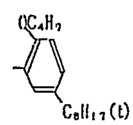 |

| No. | A | $R^1$ | $R^2$ | $R^3$ |
|---|---|---|---|---|
| 29 | 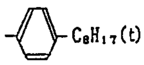 | -H | $-(CH_2)_3SO_2C_{12}H_{25}$ | 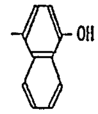 |
| 30 | 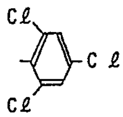 | -H | $-SC_{12}H_{25}$ | 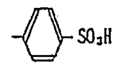 |

Examples of Compounds embodying the present invention

Examples of Compounds embodying the present invention

Formula III

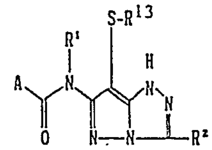

| No. | A | $R^1$ | $R^2$ | $R^{13}$ |
|---|---|---|---|---|
| 31 | 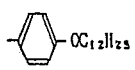 | -H | $-CH_3$ | 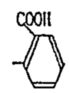 |
| 32 | 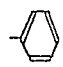 | -H | $-(CH_2)_3SO_2C_{12}H_{25}$ | 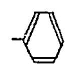 |
| 33 | 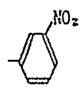 | -H | 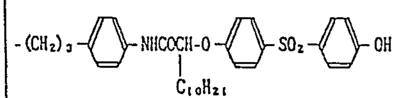 | $-CH_3$ |
| 34 | 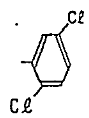 | -H | 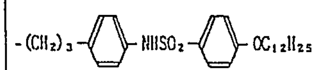 | 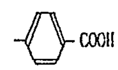 |
| 35 | 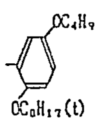 | $-CH_3$ | $-SCH_3$ | $-CH_2COOH$ |
| 36 | 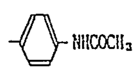 | -H | 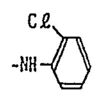 | 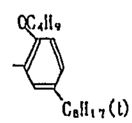 |

| No. | A | $R^1$ | $R^2$ | $R^{13}$ |
|---|---|---|---|---|
| 37 | 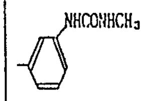 | -H | 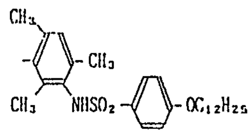 | $-CH_2CH_2COOH$ |
| 38 | 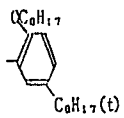 | -H | $-C_{15}H_{31}$ | 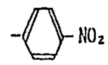 |
| 39 | 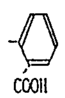 | -H | $-CHCH_2SO_2C_{12}H_{25}$<br>\|<br>$CH_3$ | 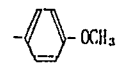 |
| 40 | 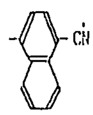 | -H | 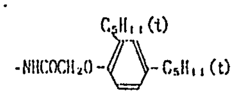 | 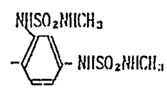 |
| 41 | 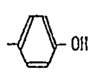 | $-COCH_3$ | $CH_3$<br>\|<br>$-CCH_2CH_2NHSO_2C_{12}H_{25}$<br>\|<br>$CH_3$ | $-CH_2CH_2N(CH_3)_2$ |
| 42 | 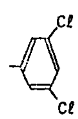 | 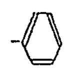 | 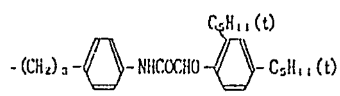 | $-CH_2CH_2OH$ |
| 43 | 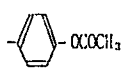 | 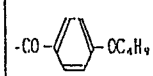 | $-SC_{12}H_{25}$ | $-CH_2CH_2SCH_2CH_2OH$ |

| No. | A | $R^1$ | $R^2$ | $R^{13}$ |
|---|---|---|---|---|
| 44 | 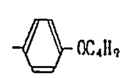 | -H | 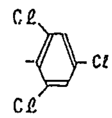 | 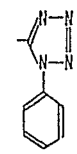 |
| 45 | 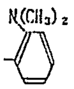 | -H | 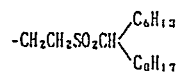 | 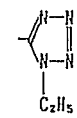 |
| 46 | 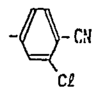 | -H | 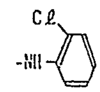 | $-CH_2CH_2OH$ |
| 47 | 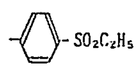 | -H | $-SCH_2CH_2COOH$ | 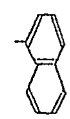 |
| 48 | 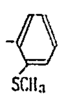 | -H | 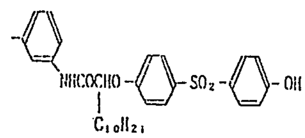 | 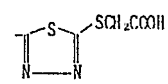 |
| 49 | 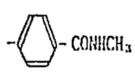 | $-C_2H_5$ | 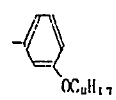 | 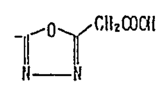 |
| 50 | 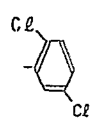 | -H | 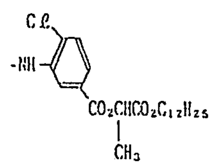 | 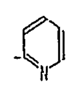 |

| No. | A | $R^1$ | $R^2$ | $R^{13}$ |
|---|---|---|---|---|
| 51 | 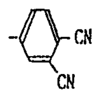 | -H | 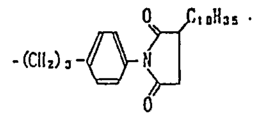 | 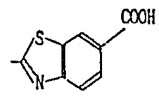 |
| 52 | 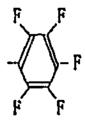 | -H | $-C_2H_5$ | 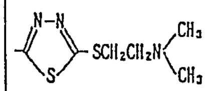 |
| 53 | 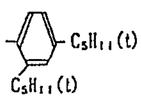 | 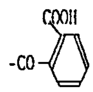 | 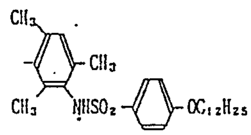 | 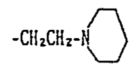 |
| 54 | 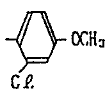 | -H | 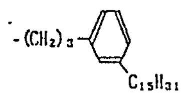 | $-CH(CH_2COOH)_2$ |
| 55 | 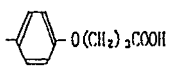 | -H | 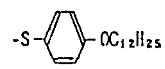 | $-CH_2CH_2OCH_2CH_2OH$ |
| 56 | 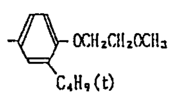 | -H | 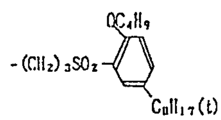 | 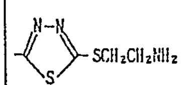 |
| 57 | 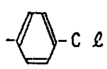 | -H | 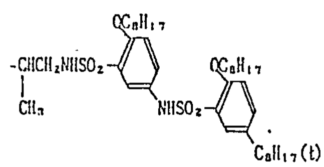 | 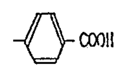 |

| No. | A | $R^1$ | $R^2$ | $R^{13}$ |
|---|---|---|---|---|
| 58 | 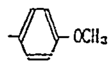 | -H | 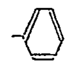 | 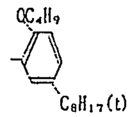 |
| 59 | 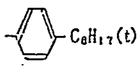 | -H | $-(CH_2)_3SO_2C_{12}H_{25}$ | 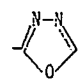 |
| 60 | 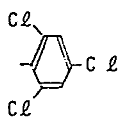 | -H | $-SC_{12}H_{25}$ | $-CH_2COOH$ |

Formula IV

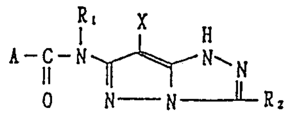

| No. | A | $R_1$ | X | $R_2$ |
|---|---|---|---|---|
| 61 | 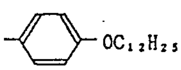 | H | Cℓ | $-CH_3$ |
| 62 | 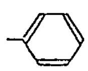 | H | Cℓ | $-(CH_2)_3SO_2C_{12}H_{25}$ |
| 63 | 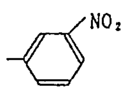 | H | Cℓ | 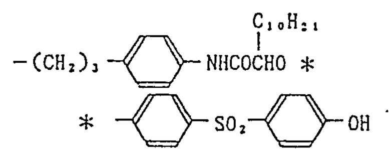 |
| 64 | 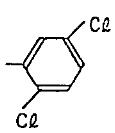 | H | Cℓ | 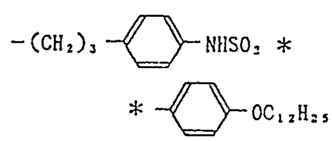 |

| No. | A | $R_1$ | X | $R_2$ |
|---|---|---|---|---|
| 65 | 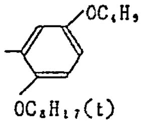 | $-CH_3$ | Cℓ | $-SCH_3$ |
| 66 | 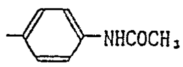 | H | Cℓ | 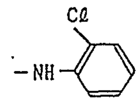 |
| 67 | 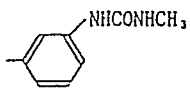 | H | Cℓ | 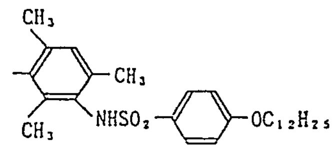 |
| 68 | 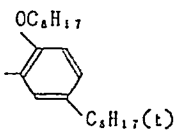 | H | Cℓ | $-C_{15}H_{31}$ |
| 69 | 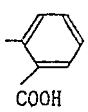 | H | Br | $-\underset{\displaystyle CH_3}{\underset{\mid}{C}}HCH_2SO_2C_{12}H_{25}$ |

| No. | A | $R_1$ | X | $R_2$ |
|---|---|---|---|---|
| 70 | 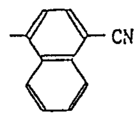 | H | Cℓ | 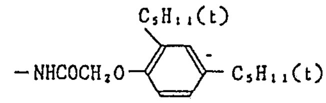 |
| 71 | 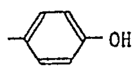 | $-COCH_3$ | Cℓ | 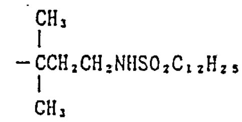 |
| 72 | 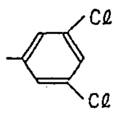 | 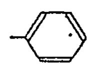 | Cℓ | 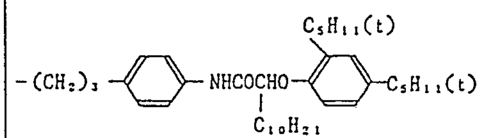 |
| 73 | 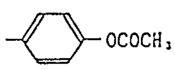 | 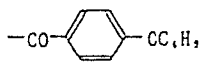 | Cℓ | $-SC_{12}H_{25}$ |
| 74 | 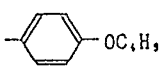 | H | Cℓ | 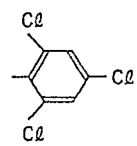 |
| 75 | 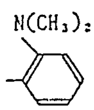 | H | Cℓ | 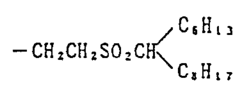 |

| No. | A | $R_1$ | X | $R_2$ |
|---|---|---|---|---|
| 76 | 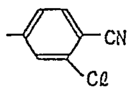 | H | Br | 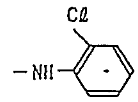 |
| 77 | 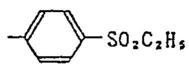 | H | Cl | $-SCH_2CH_2COOH$ |
| 78 | 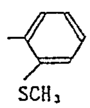 | H | Cl | 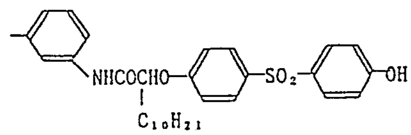 |
| 79 | 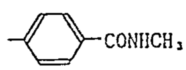 | $-C_2H_5$ | Cl | 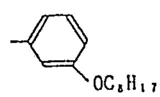 |
| 80 | 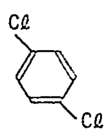 | H | I | 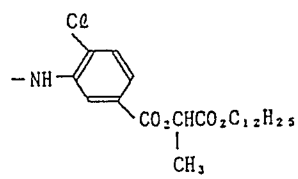 |
| 81 | 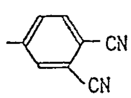 | H | Cl | 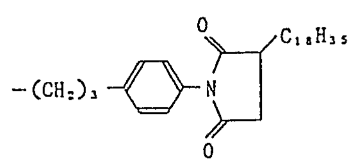 |

| No. | A | $R_1$ | X | $R_2$ |
|---|---|---|---|---|
| 82 | 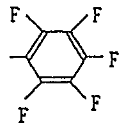 | H | Cℓ | $-C_2H_5$ |
| 83 | 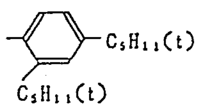 | 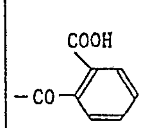 | Cℓ | 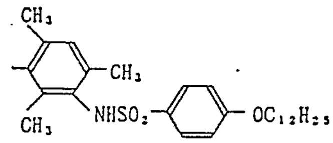 |
| 84 | 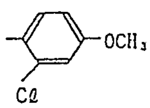 | H | Cℓ | 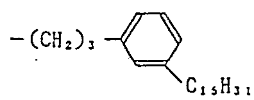 |
| 85 | 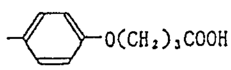 | H | I | 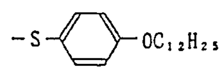 |
| 86 | 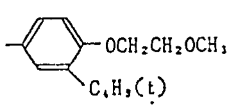 | H | Br | 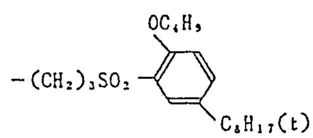 |

| No. | A | $R_1$ | X | $R_2$ |
|---|---|---|---|---|
| 87 | 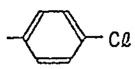 | H | Cl | 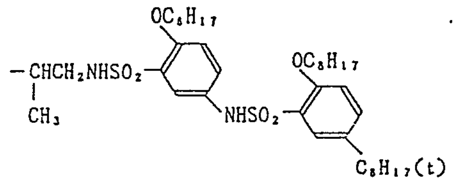 |
| 88 | 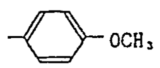 | H | Cl | 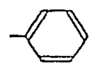 |
| 89 | 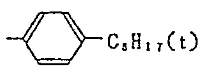 | H | Br | $-(CH_2)_3SO_2C_{12}H_{25}$ |
| 90 | 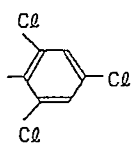 | H | Br | $-SC_{12}H_{25}$ |

A description is given next with respect to an exemplary method for synthesizing compounds as couplers embodying this invention. Procedure of Synthesis Synthesis of Compound No., 62, 1 and 32.

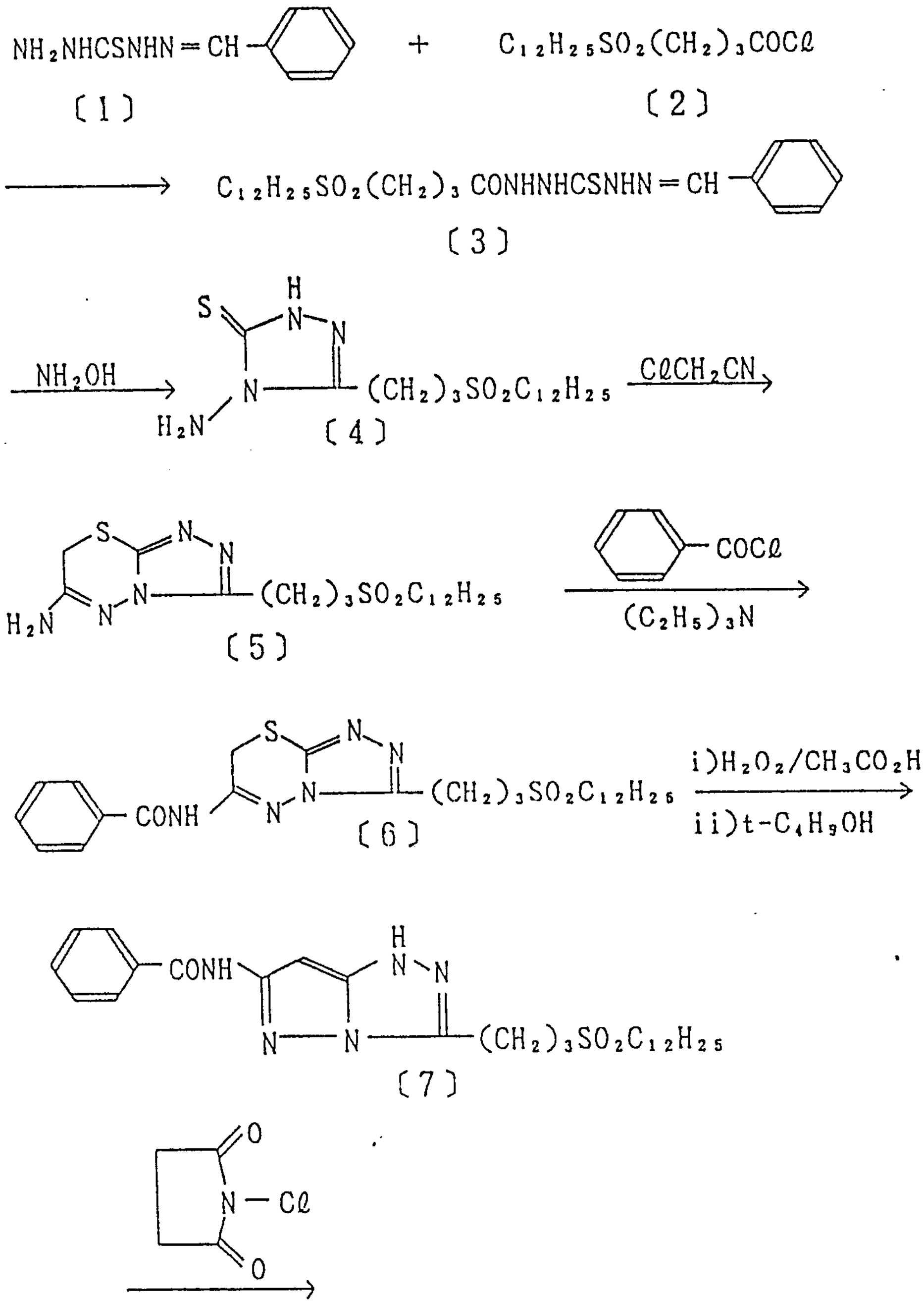

$NH_2NHCSNHN=CH-$
[1]
+
$C_{12}H_{25}SO_2(CH_2)_3COCl$
[2]
$C_{12}H_{25}SO_2(CH_2)_3CONHNHCSNHN=CH-$
[3]
$NH_2OH$
$(CH_2)_3SO_2C_{12}H_{25}$
[4]
$ClCH_2CN$
$(CH_2)_3SO_2C_{12}H_{25}$
[5]
COCl
$(C_2H_5)_3N$
CONH
$(CH_2)_3SO_2C_{12}H_{25}$
[6]
i) $H_2O_2/CH_3CO_2H$
ii) $t-C_4H_9OH$
CONH
$(CH_2)_3SO_2C_{12}H_{25}$
[7]

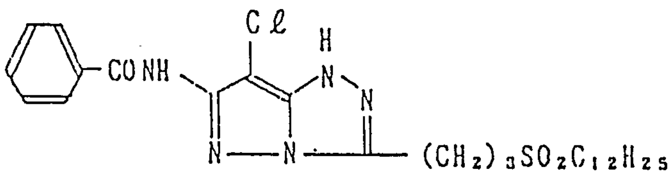

[8]

Compound No. 62

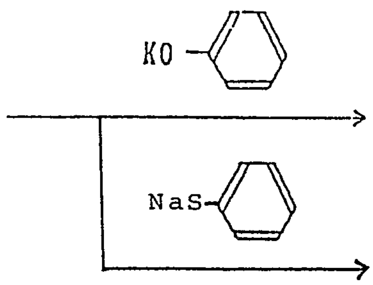

Compound No. 1

Compound No. 32

To a suspension of 31.4 g of the above-mentioned compound [I] in 320 mℓ of acetonitrile was added 18 mℓ of pyridine. Then, after increasing the temperature of the liquid to 50°C, 54.9 g of the above-mentioned compound [2] dissolved in 100 mℓ of acetonitrile was added to the liquid mixture. After stirring this mixture at the same temperature for 30 minutes under heat and subsequent cooling, a solid precipitate produced thereby was filtered off, washed with water and dried to yield 72.0 g (90%) of the above-mentioned compound [3].

Then, to 60 g of the above-mentioned compound [3] suspended in 800 mℓ of ethanol was added 20 g of 50% hydroxylamine and the liquid was heated under reflux for 7 hours. Insoluble substances were filtered out during heating, and a solid precipitate resulting from subsequent cooling was filtered off to yield the above-mentioned compound [4] in a quantity of 55.2 g (72%). Next, to a solution of 7.2 g of KOH dissolved in 1.5 ℓ of ethanol were added 50 g of the above-mentioned compound [4] and 11.5 g of chloroacetonitrile and the liquid was heated under reflux for 2 hours. Crystals precipitated by subsequent cooling were filtered off, washed with water and dried, and recrystallized in 800 mℓ of ethanol to yield the above-mentioned compound [5] in a quantity of 42.1 g (75%).

To a solution of 40.0 g of the above-mentioned compound [5] dissolved in 1 ℓ of ethyl acetate 13.1 g of triethylamine and 16.9 g of benzoylchloride were added and allowed to react at room temperature for 3 hours. A liquid product resulting from the reaction was washed with water and concentrated under reduced pressure, and the residue was recrystallized in methanol to yield the above-mentioned compound [6] in a quantity of 37.8 g (73.5%).

To a solution of 35 g of the above-mentioned compound [6] dissolved in 500 mℓ of acetic acid were added 2.4 g of tungstic acid dihydrate and 20 mℓ of 35% $H_2O_2$ and their mixture was stirred at room temperature for 6 hours. Then, their liquid reaction product was poured into iced water and, after neutralization with sodium bicarbonate water, extracted with ethyl acetate. The liquid extract was washed with water and concentrated under reduced pressure and the residue, by adding 350 mℓ of tertiary butanol thereto, was heated under reflux for one hour. The liquid reaction product was then concentrated under reduced pressure, and the residue was recrystallized in acetonitrile to yield the above-mentioned compound [7] in a quantity of 30.1 g (80%).

To a solution of 30 g of the above-mentioned compound [7] dissolved in 150 mℓ of ethyl acetate was added 9.6 g of N-chlorosuccinimide (NCS) and the liquid was heated under reflux for 30 minutes. Then, their liquid reaction product was concentrated under reduced pressure, and the residue was recrystallized in acetonitrile to yield the above-mentioned compound [8] in a quantity of 26.7 g (83%). The resulted compound is Compound No. 62.

Next, 4.8 g of phenol was dissolved in 150 mℓ of toluene, and after adding 3.4 g of KOH (in terms of 86% KOH), the liquid was refluxed by means of an ester tube with stirring in such a manner as to be continuously dehydrated for 2.5 hours. Then, a solution of 25 g of the above-mentioned compound [8] dissolved in 250 mℓ of diglyme was added dropwise thereto over a period of 30 minutes, and the liquid was refluxed further for 3.5 hours with stirring.

The liquid reaction product was cooled and the extract obtained by adding 700 mℓ of ethyl acetate was washed with water four times. The liquid extract was concentrated under reduced pressure, and the residue was recrystallized in acetonitrile finally to form Compound No. 1 of the invention in a quantity of 21.0 g (75%).

On the other hand to a solution of 25 g of the above-mentioned compound [8] dissolved in 300 mℓ of ethanol, 7.4 g of sodium sull of thiophenol was added and the liquid was heated further for 2.5 hours with refluxing undissolved material was filtered off from the resulted solution when it was hot, and the solution was concentrated under reduced pressure, and the residue was recrystallized in n-hexane/ethylacetate finally to form Compound No. 62 of the invention in a quantity of 21.2 g (72%).

The structure of these compounds respectively was identified by IR, NMR, and Mass spectrums.

A coupler of this invention is used ordinarily in the range from 1 x $10^{-3}$ to 1 mol, or preferably from 1x $10^{-2}$ to 8 x $10^{-1}$ mol, per 1 mol silver halide.

A coupler of this invention can be employed in combination with a different kind of magenta coupler insofar as the effect of this invention is not impaired.

In the practice of this invention, as the silver halide in a silver halide emulsion, use can be made of any of those in use for ordinary silver halide emulsions, such as silver bromide, silver iodo-bromide, silver iodo-chloride, silver chloro-bromide, silver chloro-iodo-bromide, silver chloride, etc. The silver halide grain can be one in which the silver halide is distributed with a uniform composition as well as one of the core/shell type in which the silver halide differs in composition between the interior and the surface layer. The silver halide grain can be one in which a latent image is mainly formed in the surface as well as one in which a latent image is mainly formed in the interior. The silver halide grain can be one which has a regular crystal shape, such as a cube, octahedron and tetradecahedron, as well as one which has an irregular crystal shape, such as a spherical or tabular crystal; the grain can be introduced arbitrarily with respect to the ratio between the [100] faces and the [111] faces; use can be made of a grain with a composite crystal shape as well as a mixture of grains varied in crystal shape. The silver halide grain should have a grain size in the range of 0.05 to 30μ, preferably in the range of 0.1 to 20μ. The grain size distribution of the silver halide emulsion is not confined to any specific pattern; use can be made of a polydisperse emulsion, i,e., a dispersion having a wide grain size range, as well as a monodispersed emulsion, i.e., a dispersion having a narrow grain size range, or a mixture of a plurality of different monodispersed emulsions; a mixture of a polydisperse emulsion and a monodispersed emulsion is also useful.

A coupler used in the practice of this invention may contain a colored coupler having a color-correcting effect and compounds which, upon coupling with the oxidized product of the developing agent, release photographically useful fragments, such as developing inhibitor, development accelerator, bleaching accelerator, developer, silver halide solvent, toner, hardener, fogging agent, antifogging agent, chemical sensitizer, spectral sensitizer, and desensitizer. In this respect, use can be made of a so-called DIR compound, which releases a developing inhibitor with development and improves the sharpness and graininess of an image.

Included in such so-called DIR compounds are one type in which a developing inhibitor is bonded directly to a coupling position and another type in which a developing inhibitor is bonded to a coupling position with a divalent group intervening therebetween so that the intramolecular nucleophilic reaction, intramolecular electron transfer reaction, etc. occurring in the group split off by the coupling cause the developing inhibitor to be released (referred to as "timing DIR compound"). Use can be made of either a developing inhibitor which diffuses well after the release or one which does not according to suitability to the purpose or the two different types jointly. Use can be made of a non-color-forming coupler (referred to also as "competitive coupler"), which performs a coupling reaction with the exidized product of an aromatic primary amine developer but does not form a dye, in combination with a dyeforming coupler.

In the practice of this invention, as a yellow coupler, the use of a known coupler based on acylacetoanilido is desirable; especially, it is of advantage to use compounds based on benzoylacetoanilido and ones based on pivaloylacetoanilido. As a cyan coupler, the use of phenol derivative or naphthol derivative is generally acceptable.

In a photosensitive material used in the practice of this invention, an anti-color fogging agent can be employed as a means to prevent loss of pureness in color caused by dislocation of the oxidized product of the developing agent or electron transferring agent between emulsion layers (between layers with the same color sensitivity and/or between layers differing in color sensitivity) or to prevent deterioration of the sharpness or to prevent graininess from becoming apparent. An image stabilizer can also be employed as a means to prevent retrogradation of the dye image; the use of the compounds described under VII - J in RD No. 17642 is desirable for this purpose. A formalin scavenger can also be employed as a means to prevent formalin from deteriorating a magenta dye-forming coupler etc. during storage. An ultraviolet absorbent can be incorporated in the hydrophilic colloidal layer, such as a protective layer and intermediate layer, as a means to prevent fogging caused by static discharge as a result of electrification of the material by friction etc. as well as to prevent UV rays from deteriorating the image.

The present invention is applicable advantageously to color negative film, color paper, color reversal film, etc.

Generally, color negative film, color paper, and color reversal film are each composed of silver halide emulsions arranged in blue-sensitive, green-sensitive, and red-sensitive layers and a nonphotosensitive hydrophilic colloidal layer; the practice of this invention is not confined to any specific arrangement of these layers on the support.

After exposure, a photosensitive material embodying this invention is subjected to color photographic processing to form the dye image. The color photographic processing comprises the steps of color developing, bleaching, fixing, and washing, and stabilizing when necessary, but the step using a bleacher and that using a fixer can be combined into a single step using a one-bath bleach-fixer, and furthermore, the steps of developíng, bleaching, and fixing can all be combined into a monobath processing using a one-bath developing-bleaching-fixing solution.

Hereunder is described the present invention in further detail by examples.

Example 1

To 0.1 mol each per 1 mol silver of the respective magenta couplers of this invention and the respective couplers for comparison, as specified in Table-1, were added tricresyl phosphate amounting to the equal quantity by weight of the couplers and ethyl acetate amounting to 3 times the quantity of the couplers by weight and the respective couplers were dissolved completely by heating up to 60°C. Each of the solutions was mixed with a preparation composed of 1,200 mℓ of 5% aqueous solution of gelatin and 120 mℓ of 5% ageous solution of Alkanol B (alkylnaphthalenesulfonate, product of Du Pont) to form a dispersed liquid preparation by emulsification with the help of an ultrasonic disperser. This dispersed liquid was added to 4 kg of green-sensitive silver iodo-bromide emulsion silver iodide content: 6 mol%) and, after adding 120 mℓ of 2% solution (water : methanol = 1:1) of 1,2-bis(vinylsulfonyl) ethane as hardener, the preparation was laid on a subbed transparent polyester base and dried finally to form samples 1 through 5 for comparison and samples 1 through 5 of this invention respectively (silver coating amounted to 20 mg/100 $cm^2$).

Each sample thus obtained was subjected to wedge exposure by an ordinary method, development by the undermentioned procedure, and measurement with respect to relative sensitivity, maximum density, and resistance to formalin, the results of which are shown in Table 1.

[Development Procedure]

| | | |
|---|---|---|
| Color developing | 38°C | 3 min. 15 sec. |
| Bleaching | 38°C | 4 min. 20 sec. |
| Washing | 38°C | 3 min. 15 sec. |
| Fixing | 38°C | 4 min. 20 sec. |
| Washing | 38°C | 3 min. 15 sec. |
| Stabilizing | 38°C | 1 min. 30 sec. |
| Drying | 47°C ± 5°C | 16 min. 30 sec. |

The process solutions used at the respective processing steps has the following compositions:

[Color developer composition]

| | |
|---|---|
| Potassium carbonate | 30.0 g |
| Sodium hydrogen carbonate | 2.5 g |
| Potassium sulfite | 5.0 g |
| Potassium bromide | 1.3 g |
| Potassium iodide | 2.0 mg |
| Hydroxylamine sulfate | 2.5 g |
| Sodium chloride | 0.6 g |
| Sodium diethylenetriamine pentaacetate | 2.5 g |
| 3-methyl-4-amino-N-ethyl-N-(β-hydroxyethyl) aniline sulfate | 4.8 g |
| Potassium hydroxide | 1.2 g |

Water is added to make the total quantity 1 ℓ and the pH is adjusted to 10.06 with potassium hydroxide or 20% sulfuric acid.

[Bleacher composition]

| | |
|---|---|
| Iron ammonium salt of ethylenediaminetetraacetic acid | 100.0 g |
| Ethylenediamine iron tetraacetate | 10.0 g |
| Ammonium bromide | 150.0 g |
| Glacial acetic acid | 40.0 mℓ |
| Sodium bromide | 10.0 g |

Water is added to make the quantity 1 ℓ and the pH is adjusted to 3.5 with aqueous ammonia or glacial acetic acid.

[Fixer composition]

| | |
|---|---|
| Ammonium thiosulfate | 180.0 g |
| Anhydrous sodium sulfite | 12.0 g |
| Sodium metabisulfite | 2.5 g |
| Disodium ethylenediamine tetraacetate | 0.5 g |
| Sodium carbonate | 10.0 g |

Water is added to make the total quantity 1 ℓ.

[Stabilizer composition]

| | |
|---|---|
| Formalin (37% aqueous solution) | 2.0 mℓ |
| Konidax (product of Konika Corp.) | 5.0 mℓ |

Water is added to make the total quantity 1 ℓ.

Table 1

| Sample | | Magenta coupler | Relative sensitivity[1)] | Maximum density | Resistance[2)] to formalin |
|---|---|---|---|---|---|
| For comparison | 1 | Compar. Coupler 1 | 100 | 1.71 | 52 |
| | 2 | Compar. Coupler 2 | 150 | 2.41 | 79 |
| | 3 | Compar. Coupler 3 | 153 | 2.40 | 77 |
| | 4 | Compar. Coupler 4 | 107 | 1.69 | 78 |
| | 5 | Compar. Coupler 5 | 165 | 2.74 | 93 |
| Invention | 1 | Compound No. 1 | 175 | 2.81 | 95 |
| | 2 | Compound No. 4 | 170 | 2.73 | 94 |
| | 3 | Compound No. 7 | 172 | 2.77 | 94 |
| | 4 | Compound No. 14 | 174 | 2.80 | 93 |
| | 5 | Compound No. 21 | 173 | 2.80 | 93 |
| | 6 | Compound No. 32 | 173 | 2.85 | 94 |
| | 7 | Compound No. 44 | 161 | 2.77 | 95 |
| | 8 | Compound No. 49 | 174 | 2.86 | 93 |
| | 9 | Compound No. 55 | 171 | 2.81 | 93 |
| | 10 | Compound No. 59 | 170 | 2.79 | 94 |
| | 11 | Compound No. 62 | 175 | 2.81 | 95 |
| | 12 | Compound No. 65 | 172 | 2.79 | 94 |
| | 13 | Compound No. 67 | 174 | 2.80 | 93 |
| | 14 | Compound No. 72 | 176 | 2.83 | 95 |
| | 15 | Compound No. 77 | 171 | 2.77 | 95 |

Note 1) The relative sensitivity is the reciprocal number of the amount of exposure giving a density of 0.10° greater than fogging density with the value of Sample 1 for comparison (Comparison coupler 1) as 100.

Note 2) In a closed vessel containing 6 mℓ of 0.9% aqueous solution of formalin and conditioned at 32°C and 62% pH the samples were made to stand for 3 days and then subjected to color developing. By way of comparison formalin-free samples were also subjected to development.

Resistance to formalin =

$$\frac{\text{Density of color image in formalin-treated sample}}{\text{Density of color image in formalin-free sample}} \times 100\ (\%)$$

Comparison Coupler 1

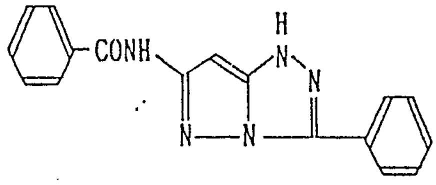

Comparison Coupler 2

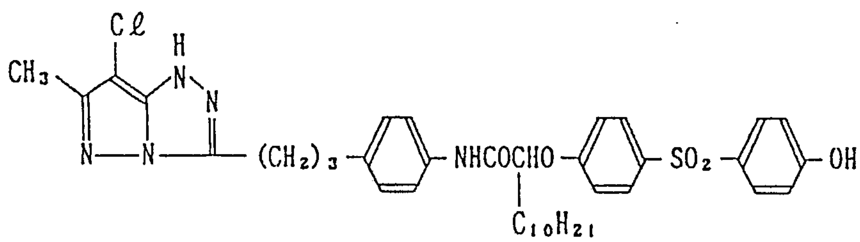

Comparison Coupler 3

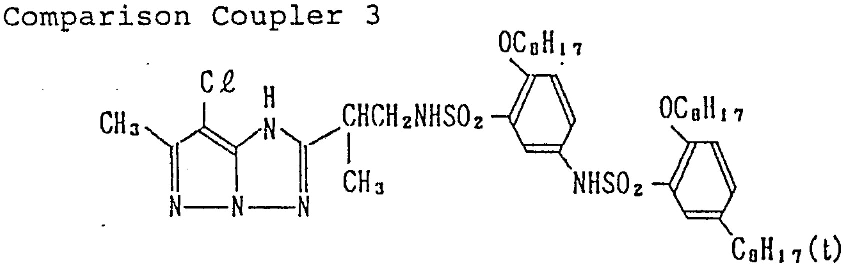

Comparison Coupler 4

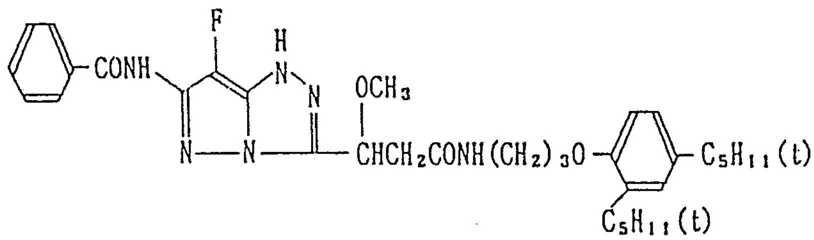

Comparison Coupler 5

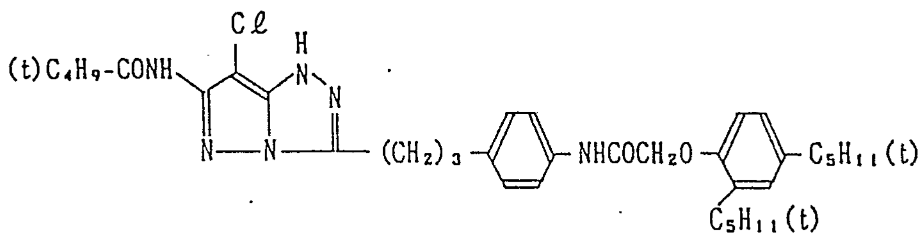

Table 1 shows that Samples of this invention as compared with Samples 1 through 5 for comparison are free from degradation in sensitivity, have good resistance to formalin, and produce dye images which have higher maximum densities.

Example 2

A multilayer color photosensitive material was prepared by laying the following layers in sequence on paper coated with polyethylene containing anatase type titanium oxide (the quantities used in the photosensitive material are expressed per 100 $cm^2$):

1) a layer containing 20 mg of gelatin, 5 mg (silver content) of blue-sensitive silver chloro-bromide emulsion (silver bromide content 80 mol%), 8 mg of yellow coupler*, 0.1 mg of 2,5-di-t-octylhydroquinone, and 3 mg of dioctylphthalate;

(2) an intermediate layer containing 12 mg of gelatin, 0.5 mg of 2,5-di-t-octylhydroquinone, 4 mg of ultraviolet absorbent*, and 2 mg of dibutylphthalate;

(3) a layer containing 18 mg of gelatin, 4 mg (silver content) green-sensitive silver chloro-bromide emulsion(silver bromide content 70 mol%), 5 mg of magenta coupler*, 2 mg of antioxident*, 0.2 mg of 2.5-di-t-octylhydroquinone, and 2.5 mg of dioctylphthalate;

(4) an intermadiate layer having the same composition as the layer (2);

(5) a layer containing 16 mg of gelatin, 4 mg (silver content) of red-sensitive silver chloro-bromide emulsion (silver bromide content 70 mol%), 3.5 mg of cyan coupler*, 0.1 mg of 2,5-di-t-octylhydroquinone, and 2.0 mg of tricresyl phosphate; and

(6) a protective layer containing 9 mg of gelatin.

* As ultraviolet absorbent in the layers (2) and (4), a mixture of UV-1 and UV-2 (mixing ratio 1:1) whose structures are shown below was employed.

* As antioxidant in the layer (3), di-t-pentylhydroquinone-di-octyl ether was employed.

With the above-mentioned layers on the support, Samples for comparison 6 and 7 and Samples of this invention 16 to 36, each consisting of a multilayer photosensitive material containing yellow coupler, magenta coupler and cyan coupler as shown in Table-2, were subjected to wedge exposure by an ordinary method, development by the undermentioned procedure, and measurement with respect to relative sensitivity, maximum density, and light fastness, the results of which are shown in Table-2. Each sample was measured with respect to magenta density after white light exposure.

[Development procedure]

| | | |
|---|---|---|
| Color developing | 38°C | 3 min. 30 sec. |
| Bleach-fixing | 33°C | 1 min. 30 sec. |
| Stabilizing (this step can be replaced by washing with water) | 25 - 30°C | 3 min. |
| Drying | 75 - 80°C | abt. 2 min. |

The process solutions used at the respective processing steps had the following compositions:

[Color developer composition]

| | |
|---|---|
| Benzyl alcohol | 15 mℓ |
| Ethylene glycol | 15 mℓ |
| Potassium sulfite | 2.0 g |
| Potassium bromide | 0.7 g |
| Potassium chloride | 0.2 g |
| Potassium carbonate | 30.0 g |
| Hydroxylamine sulfate | 3.0 g |
| Tripolyphosphate (TPPS) | 2.5 g |
| -methyl-4-amino-N-ethyl-N-(β-methanesulfonamidoethyl)aniline sulfate | 5.5 g |
| Fluorescent whitening agent (4,4′-diaminostilbenedisulfonic acid derivative) | 1.0 g |
| Pottassium hydroxide | 2.0 g |

Water is added to make the total quantity 1 ℓ and the pH is adjusted to 10.20. For the samples 30 - 36, the pH value was adjusted to 11.8.

[Bleach-fixer composition]

| | |
|---|---|
| Ferric ammonium dihydrate of ethylenediaminetetraacetic acid | 60.0 g |
| Ethylenediaminetetraacetic acid | 3.0 g |
| Ammonium thiosulfate (70% solution) | 100.0 mℓ |
| Ammonium sulfite (40% solution) | 27.5 mℓ |
| Glacial acetic acid | 10.0 mℓ |

The pH is adjusted to 7.1 with potassium carbonate or glacial acetic acid and water is added to make the total quantity 1 ℓ.

[Stabilizer composition]

| | |
|---|---|
| 5-chloro-2-methyl-4-isothiazoline-3-one | 1.0 g |

Water is added to make the total quantity 1 ℓ.

Ultraviolet absorbent

UV-1

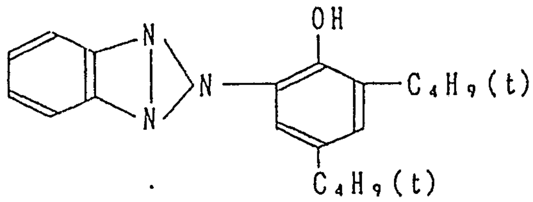

UV-2

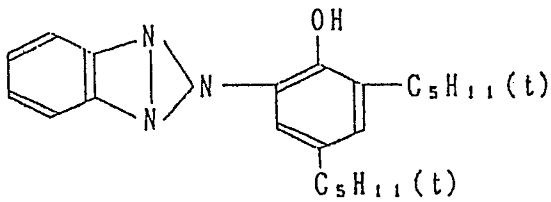

Yellow coupler

Y-1

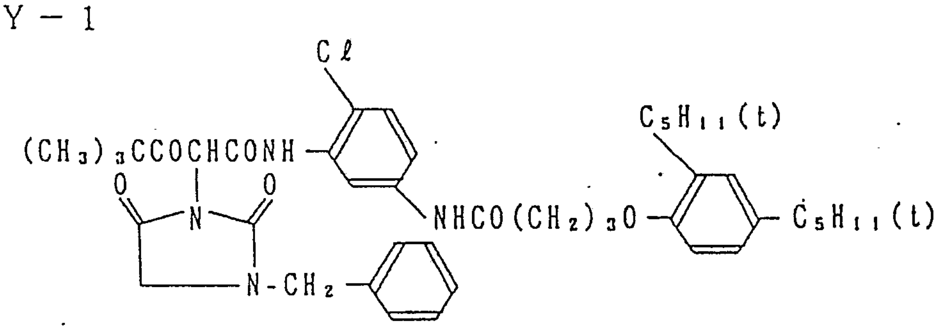

Y − 2

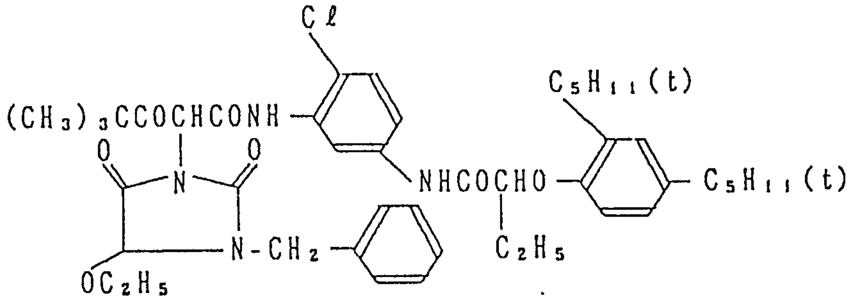

Cyan coupler

C − 1

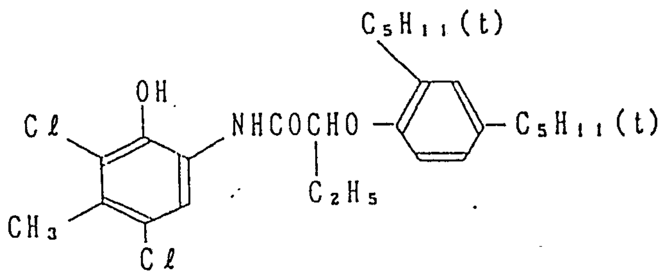

C − 2

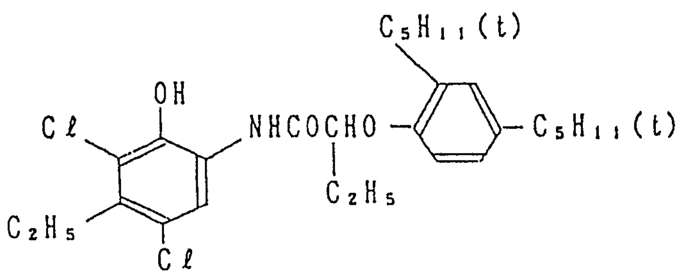

C − 3

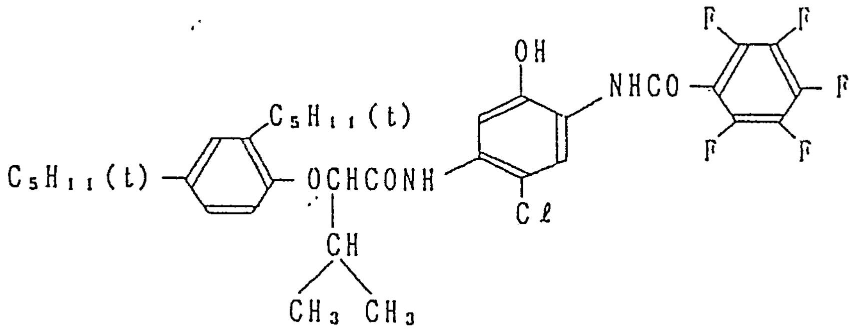

C − 4

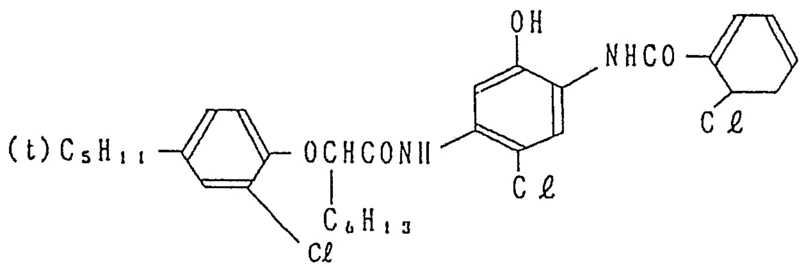

Table 2 (1/2)

| Sample | | Coupler | | | UV absorbents | Relative sensitivity[1)] | Maximu, density | Light fastness[2)] | Remarks |
|---|---|---|---|---|---|---|---|---|---|
| | | Yellow | Magenta | Cyan | | | | | |
| | | Layer (1) | Layer (3) | Layer (5) | Layers (2) & (4) | | | | |
| For comparison | 6 | Y-1 | Compar. Coupler 5 | C-1 | Not provided | 100 | 2.20 | 55 | |
| | 7 | Y-1 | Compar. Coupler 5 | C-1 | UV-1 UV-2 | 101 | 2.21 | 69 | 2 mg UV absorbent added additionally to the layer (5) |
| Invention | 16 | Y-1 | Compound No. 3 | C-1 | Not provided | 107 | 2.30 | 78 | |
| | 17 | Y-1 | Compound No. 3 | C-1 | UV-1 UV-2 | 109 | 2.33 | 90 | |
| | 18 | Y-2 | Compound No. 9 | C-2 | UV-1 UV-2 | 107 | 2.31 | 92 | |
| | 19 | Y-2 | Compound No. 9 | C-2 | UV-1 UV-2 | 96 | 2.10 | 95 | Corresponds to Sample 16 of this invention provided additionally with the same layer as Layer (2) between Layer (5) and Layer (6) |
| | 20 | Y-1 | Compound No. 15 | C-3 | UV-1 UV-2 | 105 | 2.30 | 93 | |
| | 21 | Y-1 | Compound No. 15 | C-3 | UV-1 UV-2 | 106 | 2.31 | 94 | The same as Sample 19 of this invention in layer composition |
| | 22 | Y-2 | Compound No. 29 | C-4 | UV-1 UV-2 | 106 | 2.30 | 92 | |
| | 23 | Y-1 | Compound No. 32 | C-1 | Not provided | 107 | 2.33 | 79 | |
| | 24 | Y-1 | Compound No. 32 | C-1 | UV-1 UV-2 | 109 | 2.35 | 90 | |
| | 25 | Y-2 | Compound No. 38 | C-2 | UV-1 UV-2 | 110 | 2.37 | 92 | |

Table 2 (2/2)

| Sample | | Coupler | | | UV absorbents | Relative sensitivity1) | Maximu, density | Light fastness2) | Remarks |
|---|---|---|---|---|---|---|---|---|---|
| | | Yellow | Magenta | Cyan | | | | | |
| | | Layer (1) | Layer (3) | Layer (5) | Layers (2) & (4) | | | | |
| Invention | 26 | Y-2 | Compound No. 38 | C-2 | UV-1 UV-2 | 97 | 2.20 | 96 | Corresponds to Sample 23 of this invention provided additionally with the same layer as Layer (2) between Layer (5) and Layer (6) |
| | 27 | Y-1 | Compound No. 44 | C-3 | UV-1 UV-2 | 104 | 2.30 | 93 | |
| | 28 | Y-1 | Compound No. 44 | C-3 | UV-1 UV-2 | 109 | 2.38 | 94 | The same as Sample 26 of this invention in layer composition |
| | 29 | Y-2 | Compound No. 55 | C-4 | UV-1 UV-2 | 109 | 2.40 | 93 | |
| | 30 | Y-1 | Compound No. 62 | C-1 | - | 108 | 2.31 | 70 | |
| | 31 | Y-1 | Compound No. 62 | C-1 | UV-1 UV-2 | 109 | 2.35 | 91 | |
| | 32 | Y-2 | Compound No. 70 | C-2 | UV-1 UV-2 | 108 | 2.33 | 92 | |
| | 33 | Y-2 | Compound No. 70 | C-2 | UV-1 UV-2 | 98 | 2.19 | 95 | Corresponds to Sample 32 of this invention provided additionally with the same layer as Layer (2) between Layer (5) and Layer (6) |
| | 34 | Y-1 | Compound No. 78 | C-3 | UV-1 UV-2 | 103 | 2.30 | 92 | |
| | 35 | Y-1 | Compound No. 78 | C-3 | UV-1 UV-2 | 105 | 2.28 | 95 | The same as Sample 33 of this invention in layer composition |
| | 36 | Y-2 | Compound No. 85 | C-4 | UV-1 UV-2 | 107 | 2.38 | 93 | |

Note 1) The relative sensitivity is the reciprocal number of the amount of exposure giving a density of 0.11° higher than fogging density with the value of Sample for comparison 6 (Comparison Coupler 5) as 100.

Note 2) After the color development, the samples were exposed to a xenon Fade-O-meter for 5 days so that the residual dye was determined (in %) against the initial density (D) of 100.

$$\text{Light Fastness} = \frac{\text{Density after 5 days' exposure to xenon Fade O-meter}}{1.0} \times 100\ (\%)$$

Table 2 shows that Samples of this invention as compared with Samples for comparison are free from degradation in sensitivity, have high maximum densities, and produce dye images with excellent light fastness and that such affects are enhanced by the use of ultraviolet absorbents.

Example 3

A sample 8 of a multilayer color photosensitive material for comparison was prepared by coating a support of triacetyl cellulose film with the undermentioned layers in sequence from the support. The quantities of ingredients in the color photosensitive material are shown par 1 $m^2$ unless otherwise specified. The quantities of silver halides and colloidal silver are shown in converted values representing the equivalent silver:

Layer 1

Consisting of an antihalation layer (HC), a layer of gelatin containing black colloidal silver;

Layer 2

Consisting of an intermediate layer (I.L.), a layer of gelatin containing a dispersed emulsion of 2,5-di-t-octhlhydroquinone;

Layer 3

Consisting of a layer of red-sensitive silver halide emulsion with a low sensitivity (RL), which contains a monodispersed emulsion (Emulsion I) of AgBrI (average grain size ($\bar{r}$) 0.30 μm,

| | |
|---|---|
| Ag content 6 mol%) | silver coating 1.8 g/$m^2$ |
| Sensitizing dye I | $6 \times 10^{-5}$ mol per 1 mol silver |
| Sensitizing dye II | $1.0 \times 10^{-5}$ mol per 1 mol silver |
| Cyan coupler (C-5) | 0.06 mol per 1 mol silver |
| Colored cyan coupler (CC-1) | 0.003 mol per 1 mol silver |
| DIR compound (D-1) | 0.0015 mol per 1 mol silver |
| DIR compound (D-2) | 0.002 mol per 1 mol silver |

Layer 4

Consisting of a layer of red-sensitive silver halide emulsion with a high sensitivity (RH), which contains a

monodispersed emulsion (Emulsion II) of AgBrI (average grain size ($\bar{r}$) 0.5 μm,

| | |
|---|---|
| Ag content 7.0 mol% | silver coating 1.3 g/$m^2$ |
| Sensitizing dye I | $3 \times 10^{-5}$ mole per 1 mol silver |
| Sensitizing dye II | $1.0 \times 10^{-5}$ mol per 1 mol silver |
| Cyan coupler (C-5) | 0.02 mol per 1 mol silver |
| Colored cyan coupler (CC-1) | 0.0015 mol per 1 mol silver |
| DIR compound (D-2) | 0.001 mol per 1 mol silver |

Layer 5
Consisting of an intermediate layer (I.L.), a layer of gelatin of the same composition as Layer 2

Layer 6
Consisting of a layer of green-sensitive silver halide emulsion with a low sensitivity (GL)

| | |
|---|---|
| Emulsion-I | silver coating 1.5 g/$m^2$ |
| Sensitizing dye III | $2.5 \times 10^{-5}$ mol per 1 mol silver |
| Sensitizing dye IV | $1.2 \times 10^{-5}$ mol per 1 mol silver |
| Magenta coupler (M-1) | 0.050 mol per 1 mol silver |
| Colored magenta coupler (CM-1) | 0.009 mol per 1 mol silver |
| DIR compound (D-1) | 0.0010 mol per 1 mol silver |
| DIR compound (D-3) | 0.0030 mol per 1 mol silver |

Layer 7
Consisting of a layer of green-sensitive silver halide emulsion with a high sensitivity (GH)

| | |
|---|---|
| (Emulsion-II) | silver coating 1.4 g/$m^2$ |
| Sensitizing dye III | $1.5 \times 10^{-5}$ mol per 1 mol silver |
| Sensitizing dye IV | $1.5 \times 10^{-5}$ mol per 1 mol silver |
| Magenta coupler (M-1) | 0.020 mol per 1 mol silver |
| Colored magenta coupler (CM-1) | 0.002 mol per 1 mol silver |
| DIR compound (D-3) | 0.0010 mol per 1 mol silver |

Layer 8
Consisting of a layer of yellow filter, i e. a layer of gelatin containing yellow colloidal silver and a dispersed emulsion of 2,5-di-t-octylhydroquinone

Layer 9
Consisting of a layer of blue-sensitive silver halide emulsion with a low sensitivity (BL), which contains a monodispersed emulsion (Emulsion III) of AgBrI (average grain size ($\bar{r}$) 0.48 μm,

| | |
|---|---|
| Ag content 6.0 mol%) | Silver coating 0.9 g/m$^2$ |
| Sensitizing dye V | 1.3 x $10^{-5}$ mol per 1 mol silver |
| Yellow coupler (Y-3) | 0.29 mol per 1 mol silver |

Layer 10

Consisting of a layer of blue-sensitive silver halide emulsion with a high sensitivity (BH), which contains a monodispersed emulsion (Emulsion IV) of AgBrI (average grain size ($\bar{r}$) 0.8 µm,

| | |
|---|---|
| AgI content 6 mol%) | silver coating 0.5 g/m$^2$ |
| Sensitizing dye V | 1.0 x $10^{-5}$ mol per 1 mol silver |
| Yellow coupler (Y-3) | 0.08 mol per 1 mol silver |
| DIR compound (D-2) | 0.0015 mol per 1 mol silver |

Layer 11

Consisting of a first protective layer (Pro-1), i.e., silver iodo-bromide (AgI 1 mol%, average grain size($\bar{r}$) 0.7 µm) ... silver coating 0.5 g/m$^2$ A layer of gelatin containing ultraviolet absorbents UV-1 and UV-2 (in 1:1 ratio)

Layer 12

Consisting of a second protective layer (Pro-2), i.e., a layer of gelatin containing polymethyl methacrylate grains (average diameter 1.5 µm) and formalin scavenger (HS-1).

Besides the above-mentioned ingredients, the composition of each layer included a gelatin hardener (H-1) and surface active agent.

Samples for comparison 9 through 12 and Samples 37 through 53 of this invention were prepared in the same manner as said Sample 8 for comparison, except that the magenta coupler (M-1) contained in the 6th and 7th layers of Sample 8 for comparison was replaced by different couplers as shown in Table 3.

The compounds contained as ingredients in the samples are as follows:

| | |
|---|---|
| Sensitizing dye I : | anhydro-5,5′-dichloro-9-ethyl-3,3′-di-(3-sulfo-propyl) thiacarbocyaninehy-droxide |
| Sensitizing dye II : | anhydro-9-ethyl-3,3′-di-(3-sulfopropyl)-4,5-4′,5′-dibenzo-thiacarbocyanine-hy-droxide |
| Sensitizing dye III : | anhydro-5,5′-diphenyl-9-ethyl-3,3′-di-(3-sulfo-propyl) oxycarbocyaninehy-droxide |
| Sensitizing dye IV : | anhydro-9-ethyl3,3′-di-(3-sulfopropyl)-5,6,5′,6′-diben-zooxacarbocyaninehy-droxide |
| Sensitizing dye V : | anhydro-3,3′-di-(3-sulfoprophyl)-4,5-benzo-5′-methoxythiacyanine-hydroxide |

C－5

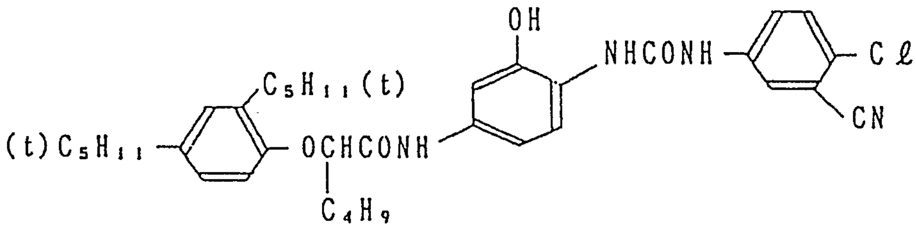

CC－1

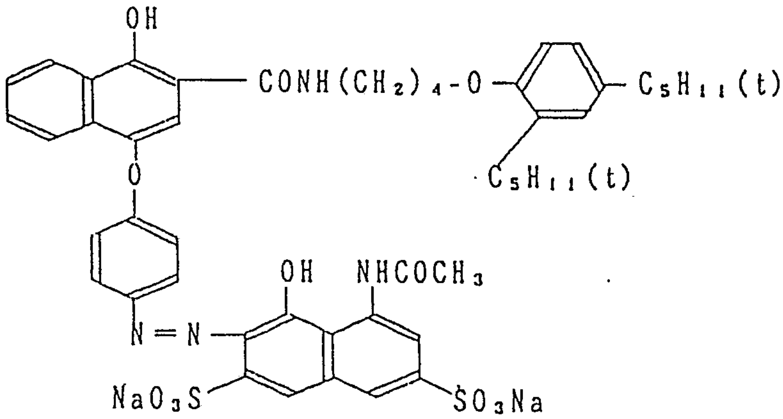

D－1

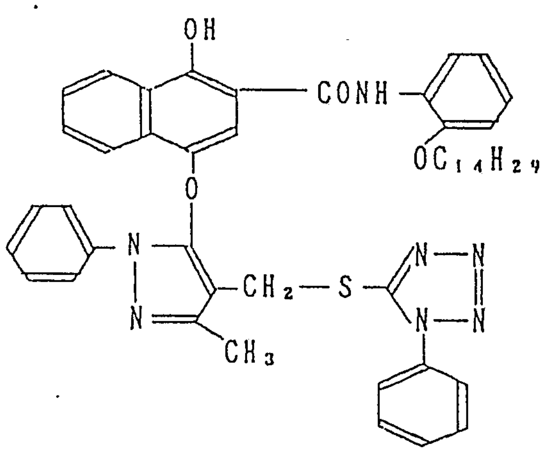

D − 2

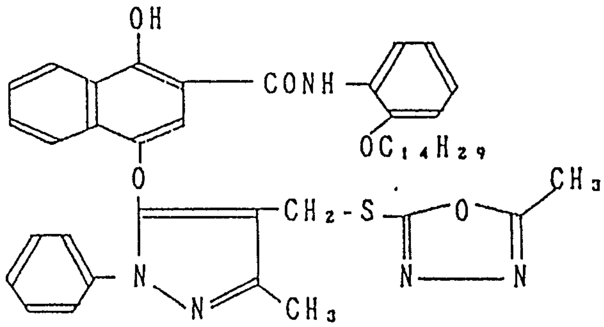

D − 3

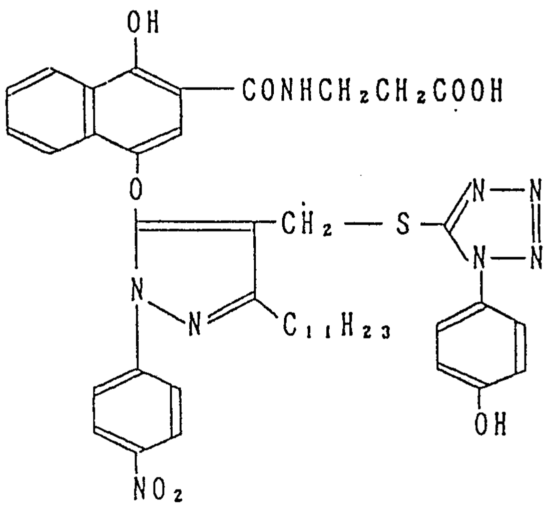

M − 1

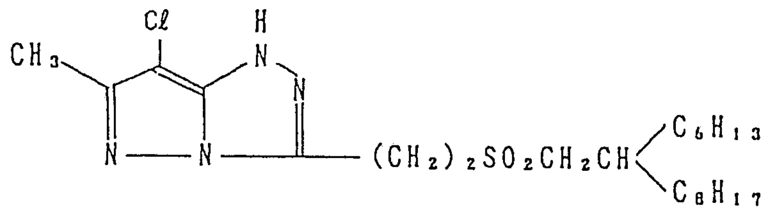

C M − 1

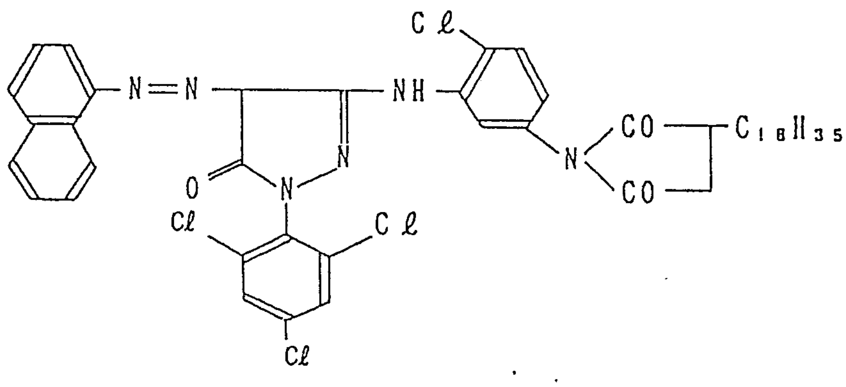

Y − 3

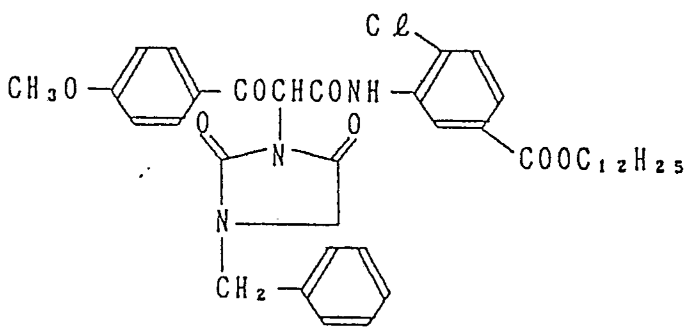

H S − 1

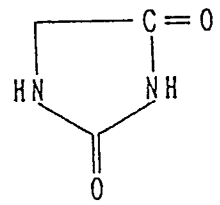

H − 1

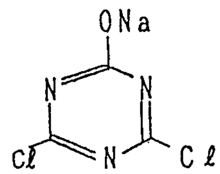

Each of one set of samples thus obtained was subjected to wedge exposure to white light and development by the undermentioned procedure [I].

[Development Procedure] (38°C)

| | |
|---|---|
| Color developing | 3 min. 15 sec. |
| Bleaching | 6 min. 30 sec. |
| Washing with water | 3 min. 15 sec. |
| Fixing | 6 min. 30 sec. |
| Washing with water | 3 min. 15 sec. |
| Stabilizing | 1 min. 30 sec. |
| Drying | |

The process solutions employed at the respective processing steps had the following compositions.

[Color developer composition]

| | |
|---|---|
| 4-amino-3-methyl-N-ethyl-N-(β-hydroxyethyl)aniline sulfate | 4.75 g |
| Anhydrous sodium sulfite | 4.25 g |
| Hydroxylamine 1/2 sulfate | 2.0 g |
| Anhydrous potassium carbonate | 37.5 g |
| Sodium bromide | 1.3 g |
| Trisodium nitrilotriacetate (monohydrate) | 2.5 g |
| Potassium hydroxide | 1.0 g |

Water is added to make the quantity 1 ℓ and the pH is adjusted to 10.06 with potassium hydroxiee or 20% sulfuric acid.

[Bleacher composition]

| | |
|---|---|
| Iron ammonium salt of ethylenediaminetetraacetic acid | 100.0 g |
| Diammonium ethylenediamine tetraacetate | 10.0 g |
| Ammonium bromide | 150.0 g |
| Glacial acetic acid | 10.0 mℓ |

Water is added to make the quantity 1 ℓ and the pH is adjusted to 6.0 with ammonia water.

[Fixer composition]

| | |
|---|---|
| Ammonium thiosulfate | 175.0 g |
| Anhydrous sodium sulfite | 8.5 g |
| Sodium metasulfite | 2.3 g |

Water is added to make the quantity 1 ℓ and the pH is adjusted to 6.0 with acetic acid.

[Stabilizer composition]

| | |
|---|---|
| Formalin (37% aqueous solution) | 1.5 mℓ |
| Konidax (product of Konika Corp.) | 7.5 mℓ |

Water is added to make the quantity 1 ℓ.

Each of another set of the same samples was subjected to the same developing procedure as [I] mentioned above, except that the pH of the color developer solution was adjusted to 9.76 in the renewed procedure [II]. Then, each sample having a dye image formed thereon was subjected to measurement of its magenta density. Table-3 shows the results specifically with respect to relative sensitivity, maximum density of the color image, resistance to formalin, and dependence on the treating condition.

The same methods of measurement were used as in Example 1 for the evaluation of the relative sensitivity and the resistance to formalin.

Table 3 (1/2)

| Sample | | Magenta coupler | Relative sensitivity[1)] | Maximum color density[1)] | Resistance to formalin[1)] | Dependence on treatment[2)] |
|---|---|---|---|---|---|---|
| For comparison | 8 | M-1 | 100 | 2.47 | 75 | 78 |
| | 9 | Compar. Coupler 1 | 78 | 1.80 | 52 | 65 |
| | 10 | Compar. Coupler 2 | 98 | 2.35 | 78 | 74 |
| | 11 | Compar. Coupler 4 | 79 | 1.85 | 85 | 80 |
| | 12 | Compar. Coupler 5 | 118 | 2.69 | 92 | 81 |
| Invention | 37 | Compound No. 4 | 123 | 2.73 | 91 | 94 |
| | 38 | Compound No. 7 | 127 | 2.81 | 94 | 93 |
| | 39 | Compound No. 13 | 120 | 2.70 | 93 | 90 |
| | 40 | Compound No. 15 | 130 | 2.77 | 94 | 92 |
| | 41 | Compound No. 17 | 125 | 2.74 | 93 | 94 |
| | 42 | Compound No. 27 | 118 | 2.65 | 90 | 88 |

Table 3 (2/2)

| Sample | | Magenta coupler | Relative sensitivity[1)] | Maximum color density[1)] | Resistance to formalin[1)] | Dependence on treatment[2)] |
|---|---|---|---|---|---|---|
| Invention | 43 | Compound No. 32 | 123 | 2.28 | 93 | 94 |
| | 44 | Compound No. 39 | 131 | 2.74 | 94 | 93 |
| | 45 | Compound No. 41 | 133 | 2.85 | 95 | 92 |
| | 46 | Compound No. 48 | 129 | 2.79 | 93 | 93 |
| | 47 | Compound No. 59 | 130 | 2.81 | 92 | 92 |
| | 48 | Compound No. 61 | 123 | 2.73 | 91 | 94 |
| | 49 | Compound No. 64 | 127 | 2.81 | 94 | 93 |
| | 50 | Compound No. 71 | 121 | 2.70 | 93 | 91 |
| | 51 | Compound No. 75 | 130 | 2.77 | 93 | 90 |
| | 52 | Compound No. 80 | 125 | 2.75 | 91 | 93 |
| | 53 | Compound No. 88 | 127 | 2.75 | 92 | 89 |

Note 1) The relative sensitivity, maximum color density (i.e., maximum density of color image), and resistance to formalin are shown in values obtained in the development procedure [I].

Note 2) Dependence on processing condition =

$$\frac{\text{Maximum density of color image formed by Development [II]}}{\text{Maximum density of color image formed by Development [I]}} \times 100\ (\%)$$

Obviously, the results shown in Table 3 prove the samples of embodying this invention to be superior to the samples 8 through 12 for comparison in not involving degradation in sensitivity, in surpassing the others in color forming property and resistance to formalin, and in retaining good color forming property even in a developer solution with a low pH.

[EFFECT OF THE INVENTION]

As will have become clear from the foregoing description, a photosensitive silver halide color photographic material provided according to this invention does not involve deterioration of the sensitivity, and has an excellent color forming property and good storability, and its photographic properties are unaffected by variations in pH of the developer solution.

## Claims

1. A silver halide color photographic material comprising a support having thereon a silver halide emulsion layer which contains a magenta coupler represented by the following formula:

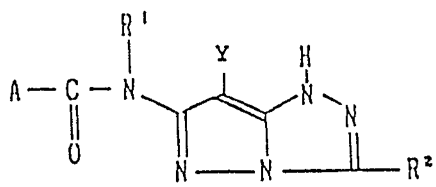

wherein $R^1$ represents a hydrogen atom, or an optionally substituted alkyl, acyl or aryl group, $R^2$ represents a hydrogen atom or a substituent group, Y represents $-O-R^3$, $-S-R^{13}$ or a chlorine, bromine or iodine atom wherein $R^3$ represents an optionally substituted aryl group, $R^{13}$ represents an optionally substituted aryl, alkyl or heterocyclic group, and A represents an optionally substituted aryl group.